# EUROPÄISCHE PATENTSCHRIFT

(11) **EP 3 131 492 B1**
(45) Veröffentlichungstag und Bekanntmachung des Hinweises auf die Patenterteilung: **14.08.2019**
(21) Anmeldenummer: 15717853.4
(22) Anmeldetag: 14.04.2015
(51) Int. Cl.: A61B 34/20

(54) **NAVIGATIONSUNTERSTÜTZUNGSSYSTEM FÜR MEDIZINISCHE INSTRUMENTE**
NAVIGATION ASSISTANCE SYSTEM FOR MEDICAL INSTRUMENTS
SYSTÈME D'ASSISTANCE À LA NAVIGATION POUR INSTRUMENTS MÉDICAUX

(30) Priorität: 15.04.2014 DE 102014207274
(43) Veröffentlichungstag der Anmeldung: 22.02.2017
(73) Patentinhaber: Fiagon AG Medical Technologies, 16761 Hennigsdorf (DE)
(72) Erfinder: KRÜGER, Timo, 13465 Berlin (DE); MUCHA, Dirk, 13467 Berlin (DE)
(74) Vertreter: Eisenführ Speiser
(86) Internationale Anmeldenummer: PCT/EP2015/058107
(87) Internationale Veröffentlichungsnummer: WO 2015/158736

(56) Entgegenhaltungen:
- EP-A1- 1 080 695
- WO-A2-2005/039391
- JP-A- 2011 036 600
- US-A1- 2005 054 895
- US-A1- 2005 085 717
- US-A1- 2009 192 519
- US-A1- 2010 249 506
- US-B1- 6 661 571

## Beschreibung

Die vorliegende Erfindung betrifft Navigationsunterstützungssysteme für medizinische Instrumente, mit einer Signalverarbeitungseinheit, die einen Eingang für Echtzeit-Videobildsignale von einem optischen Instrument, einen Eingang für Instrumentenpositionssignale sowie Zugriff auf Tomographiebilddaten besitzt und einen Ausgang zum Ausgeben von Bildsignalen, wobei die Signalverarbeitungseinheit ausgebildet ist, Ausgangsbildsignale aus den Echtzeit-Videobildsignalen, den Instrumentenpositionssignalen und den Tomographiebilddaten zu generieren. Die vorliegende Erfindung betrifft ebenfalls ein Verfahren zum Betreiben eines Navigationsunterstützungssystems.

Navigationsunterstützungssysteme der eingangs genannten Art dienen der Unterstützung invasiver chirurgischer Eingriffe durch Visualisierung und sind grundsätzlich aus dem Stand der Technik bekannt. Echtzeit-Videobildsignale können beispielsweise von einem Endoskop oder Mikroskop als optischem Instrument stammen. Instrumentenpositionssignale können durch ein optisches oder elektromagnetisches Lageerfassungssystem bereitgestellt werden, mit dem die Lage und Ausrichtung von ärztlichen Instrumenten, wie z.B. Pointern, Endoskopen oder chirurgischen Instrumenten erfasst werden können. Tomographiebilddaten werden typischerweise präoperativ angefertigt und in einer Bilddatenbank des Navigationsunterstützungssystems abgelegt.

US 2009/192519 A1 betrifft ein chirurgisches System mit einem Display zum gleichzeitigen Anzeigen einer Mehrzahl von Informationen einschließlich eines endoskopischen Bildes.

US 6,661,571 B1 offenbart ein chirurgisches mikroskopisches System, das die simultane Darstellung von Informationen verschiedener Beobachtungsquellen erlaubt.

US 2010/249506 A1 beschreibt ein medizinisches Navigationssystem bei dem die Position und die Form eines Endoskops relativ zu einem Bezugsrahmen bestimmt werden können.

US 2005/0054895 A1 offenbart ein Verfahren zur endoskopischen chirurgischen Navigation bei dem die Relativposition eines Endoskops relativ zu einer verdeckten Struktur erfasst und das Endoskop und die verdeckte Struktur entsprechend der Relativposition dargestellt werden.

WO 2005/039391 A2 beschreibt ein Verfahren zum Führen eines medizinischen Instruments, bei dem wenigstens ein intraoperatives Ultraschallbild erzeugt und ein Zielbereich auf dem Ultraschallbild dargestellt wird.

US 2005/085717 A1 offenbart ein medizinisches Navigationssystem bei dem ein intraoperatives Bild eines Patienten aufgenommen, die Position eines Instruments in einem Referenzkoordinatensystem bestimmt und ein Sichtfeld relativ zum Instrument im Referenzkoordinatensystem erzeugt wird.

EP 1 080 695 A1 betrifft eine medizinische Behandlungsvorrichtung mit einem Detektionsmittel zum Detektieren eines Körperzustands und einem Behandlungsmittel zum Behandeln des Körpers.

Der Erfindung liegt die Aufgabe zugrunde, ein Navigationsunterstützungssystem für medizinische Instrumente bereitzustellen, das eine verbesserte Unterstützung invasiver chirurgischer Eingriffe ermöglicht.

Erfindungsgemäß wird die Aufgabe gelöst durch ein Navigationsunterstützungssystem gemäß dem Anspruch 1.

Erfindungsgemäß weist das Navigationsunterstützungssystem eine Signalverarbeitungseinheit auf, die ausgebildet ist, die Ausgangsbildsignale derart zu generieren, dass die Echtzeit-Videobildsignale permanent zentral auf einer an das Navigationsunterstützungssystem angeschlossenen Bildanzeigeeinheit in einem runden oder ovalen zentralen Bildfeld eines Gesamtbildes angezeigt werden, wobei sich das zentrale Bildfeld wenigstens in einer Erstreckungsrichtung über den größten Teil, wenigstens aber mehr als die Hälfte des Gesamtbildes erstreckt. Gleichzeitig ist die Signalverarbeitungseinheit ausgebildet, die Ausgangssignale derart zu generieren, dass aus Tomographiebilddaten generierte Nebenbilder in Abhängigkeit der Instrumentenpositionssignale neben oder das zentrale Bildfeld teilweise überlagernd dargestellt werden.

Die Erfindung schließt die Erkenntnis ein, dass Navigationsunterstützungssysteme des Standes der Technik die Arbeit eines Chirurgen bei invasiven chirurgischen Eingriffen bisher nur unzureichend unterstützen, bzw. diesen sogar ablenken. Navigationsunterstützungssysteme des Standes der Technik sind nämlich ausgebildet, zunächst ein beispielsweise endoskopisches Echtzeit-Videobildsignal auf einer Bildanzeigeeinheit auszugeben und anschließend auf eine gemeinsame Visualisierung von Echtzeit-Videobildsignalen, Instrumentenpositionssignalen und Tomographiebilddaten in Form eines in typischerweise vier Sektoren geteilten Bildanzeigeeinheit umzuschalten. Neben dem Umschalteffekt ergibt sich eine nachteilige weil verkleinerte Darstellung der Echtzeit-Videobildsignale. Typischerweise wird eine zusätzliche Bildanzeigeeinheit für die Darstellung der Echtzeit-Videobildsignale bereitgestellt. Somit muss auch am Navigationsunterstützungssystem ein zweiter Ausgang zum Ausgeben von Bildsignalen vorgesehen werden, was einen schaltungstechnischen Aufwand bedeutet. Zum anderen kann im Fall einer zusätzlichen Bildanzeigeeinheit der Chirurg nicht ohne Umstände gleichzeitig sowohl die erste als auch die zweite Bildanzeigeeinheit überwachen. Diese Nachteile werden durch die erfindungsgemäßen Navigationsunterstützungssysteme vermieden.

Dadurch, dass die Signalverarbeitungseinheit erfindungsgemäß ausgebildet ist, die Ausgangsbildsignale derart zu generieren, dass die Echtzeit-Videobildsignale permanent zentral und in möglichst großem Abbildungsmaßstab auf einer an das Navigationsunterstützungssystem angeschlossenen Bildanzeigeeinheit in einem runden oder ovalen zentralen Bildfeld eines Gesamtbildes angezeigt werden, muss ein Chirurg seinen Blick während der Operation nunmehr nicht vom zentralen Bereich der Bildanzeigeeinheit abwenden.

Dadurch, dass die Signalverarbeitungseinheit weiter ausgebildet ist, die Ausgangsbildsignale derart zu generieren, dass aus Tomographiebilddaten generierte Nebenbilder in Abhängigkeit der Instrumentenpositionssignale neben oder das zentrale Bildfeld teilweise überlagernd dargestellt werden, wird das Gesamtbild in jeder Operationssituation entsprechend optimal ausgenutzt. Eine Darstellung von Nebenbildern neben dem zentralen Bildfeld kann beispielsweise ausreichend sein, wenn ein chirurgisches Instrument noch nicht in einem operativen Zielgebiet befindlich ist. Befindet sich ein chirurgisches Instrument bereits in einem operativen Zielgebiet oder nähert sich diesem an, ist eine vergrößerte oder sogar das zentrale Bildfeld überlappende Darstellung von Nebenbildern wünschenswert, da das chirurgische Instrument nunmehr noch präziser geführt werden muss.

Die Erfindung schließt hierbei die Erkenntnis ein, dass in einem oder nahe eines operativen Zielgebiets der Chirurg das chirurgische Instrument typischerweise langsamer führt oder mit diesem auch länger in einer Position verharrt als außerhalb eines operativen Zielgebiets und dass eine Lage- und/oder Geschwindigkeitsinformation(en) des chirurgischen Instrumentes für zweckmäßige Darstellung von Nebenbildern ausgewertet werden kann. Entsprechend kann die Signalverarbeitungseinheit ausgebildet sein, Instrumentenpositionssignale auszuwerten und in Abhängigkeit davon die Ausgangsbildsignale derart zu generieren, dass aus Tomographiebilddaten generierte Nebenbilder neben dem zentralen Bildfeld oder das zentrale Bildfeld teilweise überlagernd dargestellt werden.

Vorteilhafterweise ergibt sich durch das erfindungsgemäße Navigationsunterstützungssystem der technische Effekt, dass ein Navigationsunterstützungssystem nur einen Ausgang zum Ausgeben von Bildsignalen bei gleichzeitiger ablenkungsfreier, weil instrumentenpositionssignalbasierter Visualisierung von medizinischen Daten aufweisen muss.

In einer bevorzugten Ausführungsform ist die Signalverarbeitungseinheit ausgebildet, die Ausgangsbildsignale derart zu generieren, dass sich das Gesamtbild über die gesamte Fläche der Bildanzeigeeinheit erstreckt. Die Signalverarbeitungseinheit kann ausgebildet sein die Ausgangsbildsignale derart zu generieren, dass sich das zentrale Bildfeld in vertikaler Erstreckungsrichtung über mehr als die Hälfte des Gesamtbildes erstreckt. Die Signalverarbeitungseinheit kann ausgebildet sein die Ausgangsbildsignale derart zu generieren, dass sich das zentrale Bildfeld in vertikaler Erstreckungsrichtung über mehr als 60% des Gesamtbildes, bevorzugt über mehr als 80% und insbesondere mehr als 90% des Gesamtbildes erstreckt, bezogen auf eine Bildanzeigeeinheit im Querformat (Landscape). Bei einer Bildanzeigeeinheit im Hochformat (Portrait) ist dementsprechend eine horizontale Erstreckungsrichtung gemeint.

Alternativ oder zusätzlich kann die Signalverarbeitungseinheit ausgebildet sein, die Ausgangsbildsignale derart zu generieren, dass sich das zentrale Bildfeld in horizontaler Erstreckungsrichtung über mehr als die Hälfte des Gesamtbildes erstreckt. Es hat sich als vorteilhaft herausgestellt, wenn die Signalverarbeitungseinheit ausgebildet ist, die Ausgangsbildsignale derart zu generieren, dass das Gesamtbild im Breitbildformat, insbesondere im Format 16:9 angezeigt wird. Auf diese Art und Weise wird dem Chirurgen ein optimales Sichtfeld bereitgestellt.

Bevorzugt ist die Signalverarbeitungseinheit ausgebildet, die Ausgangsbildsignale derart zu generieren, dass als Nebenbilder axiale, sagittale und coronare Schichten und/oder Schnitte, insbesondere von präoperativ aufgenommenen Tomographiebilddaten auf eine Bildanzeigeeinheit ausgegeben werden können. Dazu kann die Signalverarbeitungseinheit ausgebildet sein, Tomographiebilddaten aus einer Bilddatenbank auszulesen und diese entsprechend an ihrem Ausgang zum Ausgeben von Bildsignalen bereitzustellen. Vorteilhafterweise ist die Signalverarbeitungseinheit ausgebildet, die Ausgangsbildsignale derart zu generieren, dass das der unteren linken Ecke des Gesamtbildes nächstliegende Nebenbild einen axialen Schnitt eines Tomographiebildes, das der oberen linken Ecke des Gesamtbildes nächstliegende Nebenbild einen sagittalen Schnitt eines Tomographiebildes und das der oberen rechten Ecke des Gesamtbildes nächstliegende Nebenbild einen coronaren Schnitt eines Tomographiebildes zeigt. Bevorzugt ist die Signalverarbeitungseinheit ausgebildet, die Ausgangsbildsignale derart zu generieren, dass die Nebenbilder und/oder ein Hilfsbild und das zentrale Bildfeld im Sinne einer dichtesten Kreispackung auf dem Gesamtbild dargestellt werden, insbesondere, wenn die Nebenbilder das zentrale Bildfeld nicht überlappen.

Es hat sich als vorteilhaft erwiesen, wenn die Signalverarbeitungseinheit weiterhin ausgebildet ist, die Ausgangsbildsignale derart zu generieren, dass ein Hilfsbild neben oder das zentrale Bildfeld teilweise überlagernd dargestellt wird. Das Hilfsbild kann Koordinaten, Hinweise, aus tomografischen Daten konstruierte Darstellungen oder ähnliches anzeigen. Die Signalverarbeitungseinheit kann ausgebildet sein die Ausgangsbildsignale derart zu generieren, dass ein oder mehrere Nebenbilder und/oder das Hilfsbild axial zu einem Arbeitspunkt des chirurgischen Instruments, der im zentralen Bildfeld angezeigt wird, angeordnet sind.

In einer besonders vorteilhaften Ausgestaltung ist die Signalverarbeitungseinheit ausgebildet die Ausgangsbildsignale derart zu generieren, dass die Nebenbilder nur dargestellt sind, wenn das Navigationsunterstützungssystem in einem Navigationsmodus ist, d.h. insbesondere wenn ein chirurgisches Instrument, das mit dem Navigationsunterstützungssystem verbunden ist, auch tatsächlich navigiert wird. Anderenfalls, beispielsweise in einem Vorbereitungsmodus, bei dem lediglich beispielsweise ein Endoskop eingeführt nicht aber ein chirurgisches Instrument navigiert wird, kann die Signalverarbeitungseinheit ausgebildet sein die Ausgangsbildsignale derart zu generieren, dass die Nebenbilder ausgeblendet sind. Vorteilhaft sind die Nebenbilder maskiert um einen Chirurgen nicht abzulenken. Ein Umschalten zwischen einem Navigationsmodus und einem Vorbereitungsmodus kann beispielsweise auf Knopfdruck oder auch automatisch erfolgen, sobald ein Chirurg das zu navigierende Instrument aufnimmt oder ablegt. Zu diesem Zweck kann beispielsweise ein Instrumentenpositionssignal verwendet werden.

Um adäquat auf intraoperative Ereignisse reagieren zu können, hat es sich als vorteilhaft herausgestellt, wenn die Signalverarbeitungseinheit ausgebildet ist die Ausgangsbildsignale derart zu generieren, dass die Position der Nebenbilder im Gesamtbild variabel ist. So kann/können ein oder mehrere Nebenbilder mit besonders wichtigem Inhalt näher in den Aufmerksamkeitsfokus des Chirurgen verschoben werden. Insbesondere kann/können auf Benutzeraktion hin ein oder mehrere Nebenbilder im Bereich des zentralen Bildfeldes angezeigt werden. Eine Benutzeraktion kann zum Beispiel ein zeitliches Verharren mit dem Instrument oder auch eine definierte Bewegung des Instruments sein.

Ebenfalls kann es bei wichtigem Inhalt eines Nebenbilds erforderlich sein, dieses vergrößert darzustellen. Dementsprechend ist die Signalverarbeitungseinheit derart ausgebildet, dass ein oder mehrere Nebenbilder vergrößert dargestellt werden. Die Position und/oder die Größe eines oder mehrerer Nebenbilder können auch derart variabel sein, dass das zentrale Bildfeld überlappt wird. Alternativ können die Position und/oder die Größe eines oder mehrerer Nebenbilder im Gesamtbild fix sein. Um eine weiter verbesserte Visualisierung zu ermöglichen, kann die Signalverarbeitungseinheit derart ausgebildet sein, dass wenn Nebenbilder im Bereich des zentralen Bildfeldes oder dieses überlappend angezeigt werden, dies derart erfolgt, dass keine Zielstrukturen und/oder Abbildungen eines Instruments verdeckt werden.

Es hat sich als vorteilhaft herausgestellt, wenn die Signalverarbeitungseinheit ausgebildet ist, ein aufgenommenes Instrument repräsentierende Signalteile der Echtzeit-Videobildsignale zu erkennen. Die Signalverarbeitungseinheit kann insbesondere mittels einer Mustererkennung ein Instrument auf einem endoskopischen und/oder mikroskopischen Echtzeit-Videobildsignal erkennen und/oder das Instrument in schematischer Darstellung auf einer Bildanzeigeeinheit zur Anzeige bringen. Die Signalverarbeitungseinheit kann ebenfalls ausgebildet sein, Gewebestrukturen zu erkennen.

Es hat sich als vorteilhaft herausgestellt, wenn die Signalverarbeitungseinheit ausgebildet ist, die Ausgangsbildsignale derart zu generieren, dass ein aufgenommenes Instrument repräsentierende Signalteile der Echtzeit-Videobildsignale bei Annäherung des Instruments an eine kritische Struktur und/oder Zielstruktur, insbesondere bei Annäherung an eine vorgebbare Position, optisch hervorgehoben werden.

Erfindungsgemäß wird die Aufgabe ebenfalls durch ein Verfahren zum Betreiben eines vorbeschriebenen Navigationsunterstützungssystems gemäß dem Anspruch 12 gelöst.

Das Verfahren zum Betreiben eines vorbeschriebenen Navigationsunterstützungssystems umfasst den Schritt:
- Generieren von Ausgangsbildsignalen aus Echtzeit-Videobildsignalen, Instrumentenpositionssignalen und Tomographiebilddaten derart, dass die Echtzeit-Videobildsignale permanent zentral auf einer an das Navigationsunterstützungssystem angeschlossenen Bildanzeigeeinheit in einem runden oder ovalen zentralen Bildfeld eines Gesamtbildes angezeigt werden, wobei sich das zentrale Bildfeld wenigstens in einer Erstreckungsrichtung über mehr als die Hälfte des Gesamtbildes erstreckt, wobei aus Tomographiebilddaten generierte Nebenbilder in Abhängigkeit der Instrumentenpositionssignale neben oder das zentrale Bildfeld teilweise überlagernd dargestellt werden.

Das erfindungsgemäße Verfahren kann Verfahrensschritte aufweisen, die den bezüglich des Navigationsunterstützungssystem erläuterten Vorrichtungsmerkmalen entsprechen - und umgekehrt. Ist also beispielsweise bezüglich des Navigationsunterstützungssystems ausgeführt, dass die Signalverarbeitungseinheit ausgebildet ist, die Ausgangsbildsignale derart zu generieren, dass das Gesamtbild im Breitbildformat, insbesondere im Format 16:9 angezeigt wird, so ist ebenfalls der Verfahrensschritt offenbart: - Generieren der Ausgangsbildsignale durch die Signalverarbeitungseinheit derart, dass das Gesamtbild im Breitbildformat, insbesondere im Format 16:9 angezeigt wird.

Ausführungsbeispiele der Erfindung werden nun nachfolgend anhand der Zeichnungen beschrieben. Weitere Vorteile, Merkmale und Einzelheiten der Erfindung ergeben sich aus der nachfolgenden Beschreibung der bevorzugten Ausführungsbeispiele sowie anhand der Zeichnungen; diese zeigen in:
- Fig. 1: eine schematische Darstellung eines Navigationsunterstützungssystems, das an eine Bildanzeigeeinheit und zwei Instrumente angeschlossen ist;
- Fig. 2: eine schematische Darstellung eines Bildfeldes mit drei Nebenbildern;
- Fig. 3: eine schematische Darstellung eines Bildfeldes mit drei Nebenbildern und einem Hilfsbild;
- Fig. 4: eine schematische Darstellung zweier weiterer Bildfelder;
- Fig. 5: eine schematische Darstellung eines Bildfeldes mit dargestelltem Arbeitspunkt eines chirurgischen Instrumentes;
- Fig. 6: eine schematische Darstellung eines Bildfeldes mit drei Nebenbildern und einem Hilfsbild, wobei zwei Nebenbilder vergrößert sind und das zentrale Bildfeld überlappen;
- Fig. 7: eine schematische Darstellung eines Bildfeldes mit einem hervorgehobenen Arbeitspunkt eines chirurgischen Instruments.
- Fig. 8: eine schematische Darstellung eines Bildfeldes mit drei Nebenbildern und einem Hilfsbild angeordnet im Sinne einer dichtesten Kreispackung.

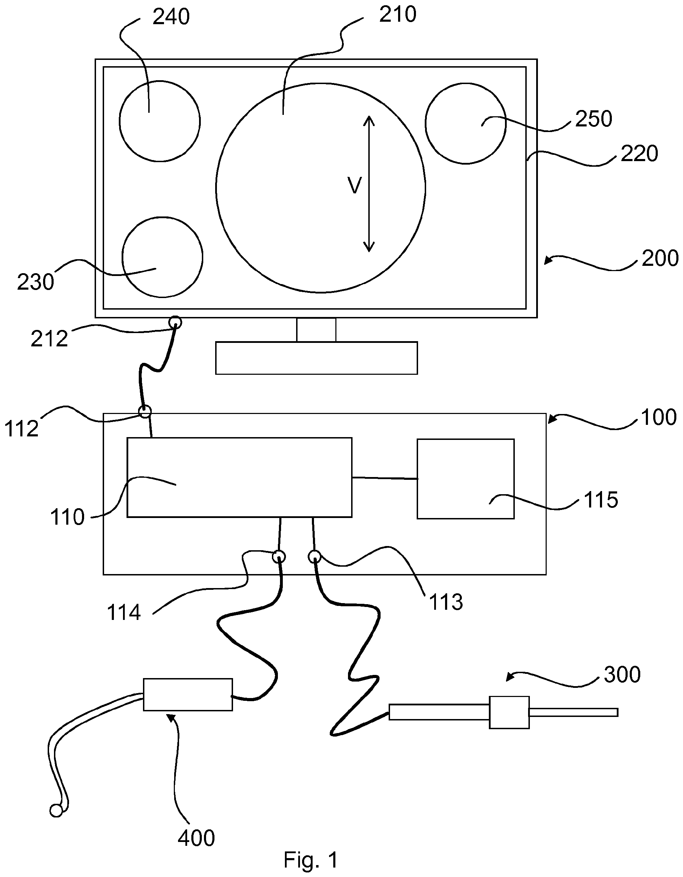

In Fig. 1 gezeigt sind ein Navigationsunterstützungssystem 100, eine Bildanzeigeeinheit 200, ein als Pointer ausgebildetes chirurgisches Instrument 300 und ein als Endoskop ausgebildetes optisches Instrument 400. Das Navigationsunterstützungssystem 100 weist eine Signalverarbeitungseinheit 110 auf, die wiederum einen Eingang 114 für Echtzeit-Videobildsignale von dem optischen Instrument 400, einen Eingang 113 für Instrumentenpositionssignale sowie Zugriff auf Tomographiebilddaten 115 besitzt und einen Ausgang 112 zum Ausgeben von Bildsignalen. Am Eingang 114 für Echtzeit-Videobildsignale ist das optisches Instrument 400 und am Eingang 113 für Instrumentenpositionssignale das chirurgisches Instrument 300 angeschlossen. Die Bildanzeigeeinheit 200 ist am Ausgang 112 zum Ausgeben von Bildsignalen angeschlossen. Die Signalverarbeitungseinheit 110 ist ausgebildet, Ausgangsbildsignale aus den Echtzeit-Videobildsignalen, den Instrumentenpositionssignalen und den Tomographiebilddaten 115 zu generieren. Diese Ausgangsbildsignale werden vorliegend auf der Bildanzeigeeinheit 200 in Form eines Gesamtbildes 220, das ein Bildformat 16:9 aufweist, angezeigt. Das Gesamtbild 220 erstreckt sich über die gesamte Fläche der Bildanzeigeeinheit 200.

Wie weiter aus Fig. 1 ersichtlich ist, ist die Signalverarbeitungseinheit 110 ausgebildet, die Ausgangsbildsignale derart zu generieren und auf der Bildanzeigeeinheit 200 anzuzeigen, dass die Echtzeit-Videobildsignale permanent zentral in einem runden zentralen Bildfeld 210 des Gesamtbildes 220 angezeigt werden. Gezeigt ist, dass sich das zentrale Bildfeld 210 in vertikaler Erstreckungsrichtung V über mehr als die Hälfte des Gesamtbildes 220 erstreckt. Neben dem zentralen Bildfeld 210 sind aus Tomographiebilddaten Instrumentenpositionssignale generierte Nebenbilder 230, 240, 250 dargestellt, die vorliegend das zentrale Bildfeld 220 nicht überlagern. Das der unteren linken Ecke des Gesamtbildes 220 nächstliegende Nebenbild 230 zeigt einen axialen Schnitt eines Tomographiebildes, das der oberen linken Ecke des Gesamtbildes 220 nächstliegende Nebenbild 240 zeigt einen sagittalen Schnitt eines Tomographiebildes und das der oberen rechten Ecke des Gesamtbildes 220 nächstliegende Nebenbild 250 zeigt einen coronaren Schnitt eines Tomographiebildes. Mit anderen Worten ist die Signalverarbeitungseinheit 110 ausgebildet, die Ausgangsbildsignale derart zu generieren, dass in der unteren linken Ecke des Gesamtbildes 220 ein axialer Schnitt, in der oberen linken Ecke ein sagittaler Schnitt und in der oberen rechten Ecke des Gesamtbildes ein coronarer Schnitt eines Tomographiebildes dargestellt wird. Das zentrale Bildfeld 210 und die Nebenbilder 230, 240, 250 weisen eine fixe Ausdehnung und Position auf dem Gesamtbild 220 auf. Aus Gründen der Einfachheit sind vorliegend die tatsächlichen Bildinhalte der jeweiligen Schnitte nicht gezeigt.

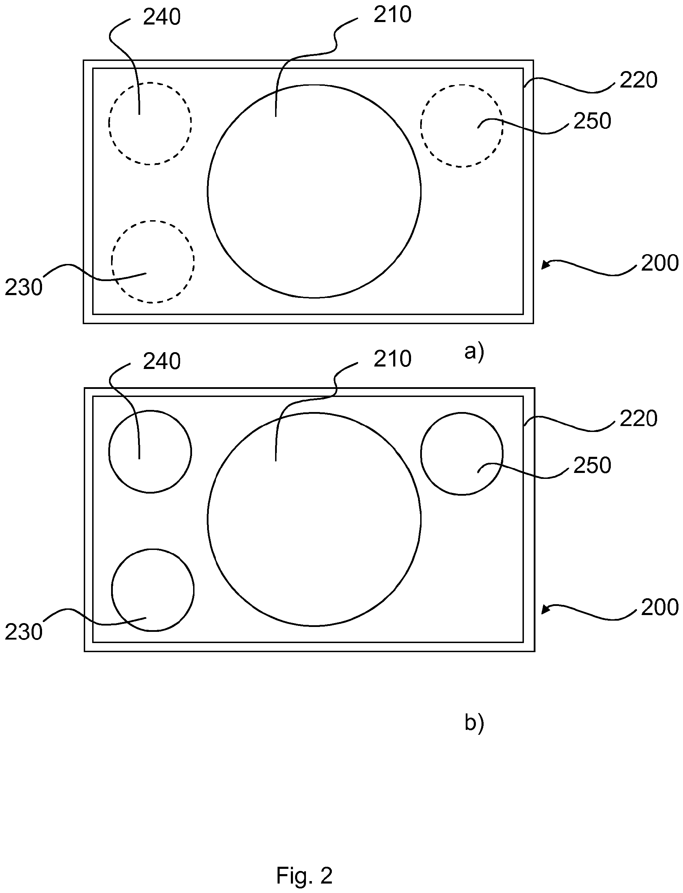

Fig. 2 zeigt ebenfalls eine Bildanzeigeeinheit 200 mit einem Gesamtbild 220. Sowohl in Fig. 2a) als auch in Fig. 2b) werden Echtzeit-Videobildsignale des (nicht gezeigten) optischen Instruments permanent zentral in einem runden zentralen Bildfeld 210 des Gesamtbildes 220 angezeigt. Wie auch in den anderen Figuren ist eine permanente Darstellung im Betrieb durch eine durchgezogene Kontur dargestellt. Fig. 2a) zeigt nun einen Vorbereitungsmodus in dem die Nebenbilder 230, 240, 250 maskiert sind um den Chirurgen beim Einführen eines (nicht gezeigten) optischen Instruments nicht zu stören. Die (nicht gezeigte) Signalverarbeitungseinheit generiert die Ausgangsbildsignale also derart, dass die Nebenbilder 230, 240, 250 nicht angezeigt bzw. maskiert sind. Fig. 2b) zeigt wiederum einen Navigationsmodus bei dem die (nicht gezeigte) Signalverarbeitungseinheit die Ausgangsbildsignale derart generiert, dass die Nebenbilder 230, 240, 250 angezeigt bzw. nicht maskiert sind. Wie auch in den anderen Figuren ist eine Maskierung im Betrieb durch eine gestrichelte Kontur dargestellt.

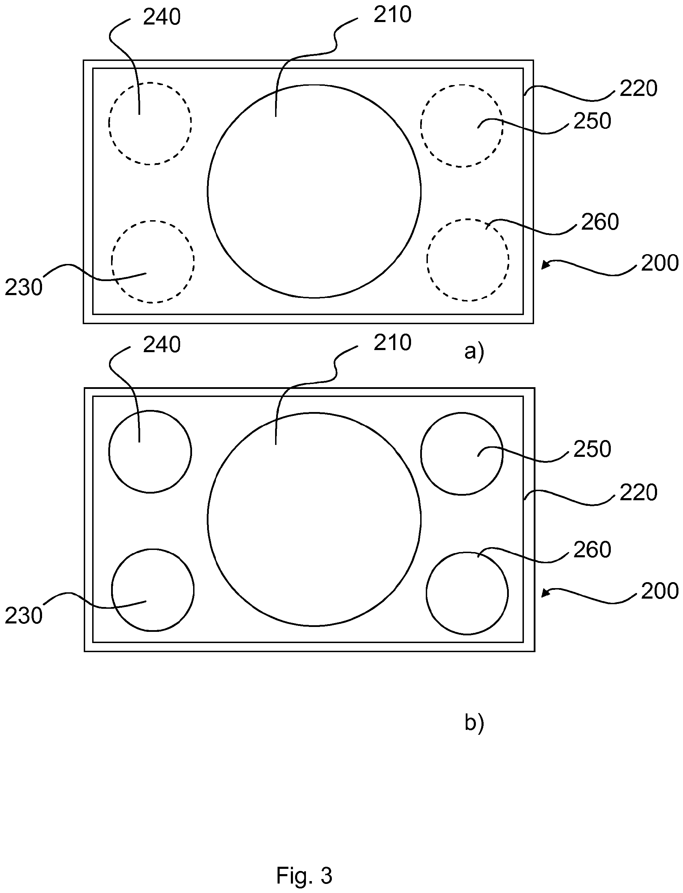

Fig. 3 zeigt auch eine Bildanzeigeeinheit 200 mit einem Gesamtbild 220. Sowohl in Fig. 3a) als auch in Fig. 3b) werden Echtzeit-Videobildsignale des (nicht gezeigten) optischen Instruments permanent zentral in einem runden zentralen Bildfeld 210 des Gesamtbildes 220 angezeigt. Fig. 3b) zeigt einen Navigationsmodus bei dem die (nicht gezeigte) Signalverarbeitungseinheit die Ausgangsbildsignale derart generiert, dass die Nebenbilder 230, 240, 250 angezeigt bzw. nicht maskiert sind. Zusätzlich generiert die (nicht gezeigte) Signalverarbeitungseinheit die Ausgangsbildsignale derart, dass am nächsten zur unteren rechten Ecke des Gesamtbildes 220 ein aus tomografischen Daten generiertes Hilfsbild 260 dargestellt wird. Im Vorbereitungsmodus, der in Fig. 3a) gezeigt ist, sind sowohl die Nebenbilder 230, 240, 250 als auch das Hilfsbild 260 maskiert. Die (nicht gezeigte) Signalverarbeitungseinheit generiert die Ausgangsbildsignale also derart, dass die Nebenbilder 230, 240, 250 und Hilfsbild 260 nicht angezeigt bzw. maskiert sind. Das zentrale Bildfeld 210, die Nebenbilder 230, 240, 250 und das Hilfsbild 260 weisen eine fixe Ausdehnung und Position auf dem Gesamtbild 220 auf.

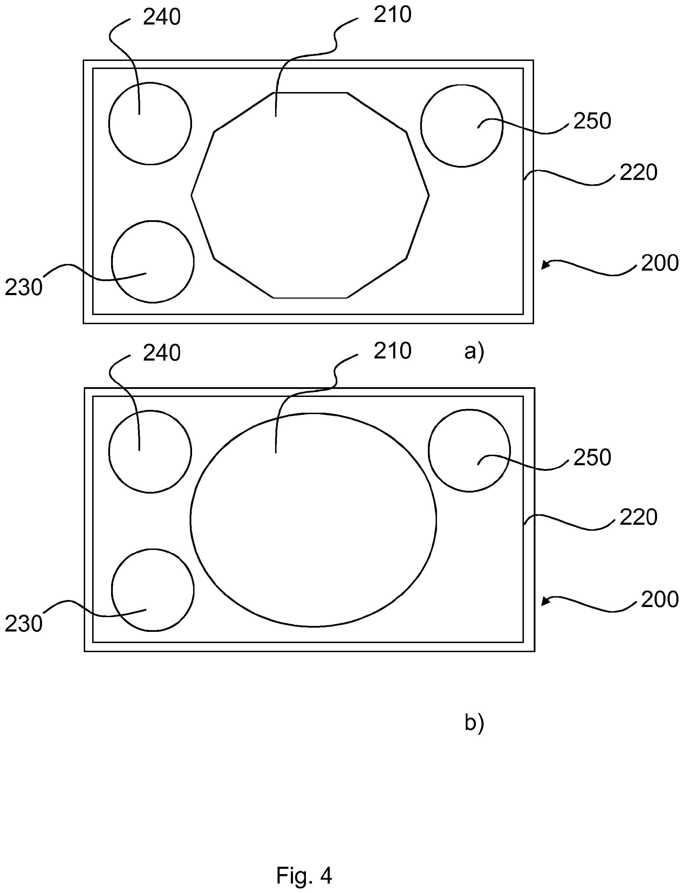

Fig. 4 zeigt ebenfalls eine Bildanzeigeeinheit 200 mit einem Gesamtbild 220. In Fig. 4a) werden Echtzeit-Videobildsignale des (nicht gezeigten) optischen Instruments permanent zentral in einem im Wesentlichen runden zentralen Bildfeld 210 des Gesamtbildes 220 angezeigt. In Fig. 4b) werden Echtzeit-Videobildsignale des (nicht gezeigten) optischen Instruments permanent zentral in einem ovalen zentralen Bildfeld 210 des Gesamtbildes 220 angezeigt. In beiden Unterfiguren ist gezeigt, dass sich das zentrale Bildfeld 210 in vertikaler Erstreckungsrichtung V über mehr als die Hälfte des Gesamtbildes 220 erstreckt.

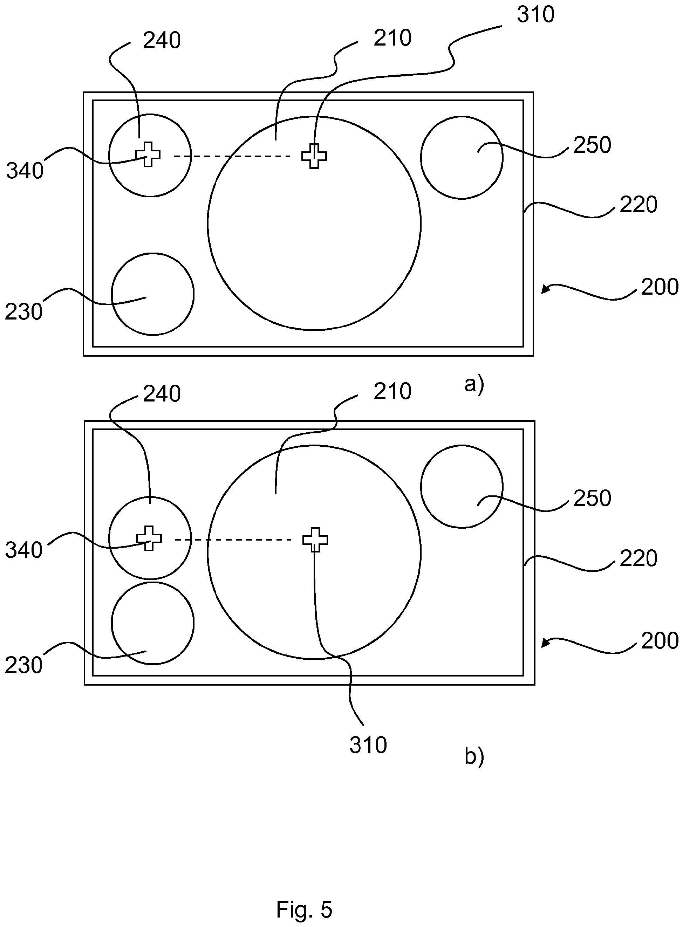

Ein Navigationsunterstützungssystem dessen (nicht gezeigte) Signalverarbeitungseinheit die Ausgangsbildsignale derart generiert, dass die Position der Nebenbilder 230, 240, 250 im Gesamtbild 220 variabel ist, ist in Fig. 5 gezeigt. Die Darstellung des Gesamtbildes 220 in Fig. 5a) entspricht derer in Fig. 1 und Fig. 2b) mit dem Unterschied, dass in Fig. 5a) im zentralen Bildfeld 210 ein zentrales Fadenkreuz 310 und im sagittalen Nebenbild 240 ein sagittales Fadenkreuz 340 gezeigt ist, wobei die Fadenkreuze 310, 340 jeweils den Arbeitspunkt des (nicht gezeigten) chirurgischen Instruments 300 repräsentiert. Durch die gestrichelte Linie wird angedeutet, dass das zentrale Fadenkreuz 310 und das sagittale Fadenkreuz 340 horizontal auf gleicher Achse auf dem Gesamtbild 220 durch das Navigationsunterstützungssystem angezeigt werden. Wird nun das (nicht gezeigte) chirurgische Instrument 300, wie in Fig. 5b) gezeigt, in eine andere Lage gebracht, so verschiebt sich das zentrale Fadenkreuz 310 auf dem fixen zentralen Bildfeld 210, vorliegend nach unten. Da in dem Ausführungsbeispiel der Fig. 5 eine gemeinsame horizontale Lage des zentralen Fadenkreuzes 310 und des sagittalen Fadenkreuzes 340 erwünscht ist - dies um einem Chirurgen eine intuitivere Bilddarstellung anzubieten - generiert die (nicht gezeigte) Signalverarbeitungseinheit die Ausgangsbildsignale derart, dass die Position des sagittalen Nebenbildes 240 bezüglich seiner Ausgangslage verschoben wird so dass das sagittale Fadenkreuz 340 wieder auf eine gemeinsame horizontale Achse mit dem zentralen Fadenkreuz 310 gelangt. Im Ausführungsbeispiel der Fig. 5 ist nur das sagittale Nebenbild 240 als positionsvariabel dargestellt. Selbstverständlich können auch das axiale Nebenbild 230 und/oder das coronare Nebenbild 250 entsprechend positionsvariabel sein.

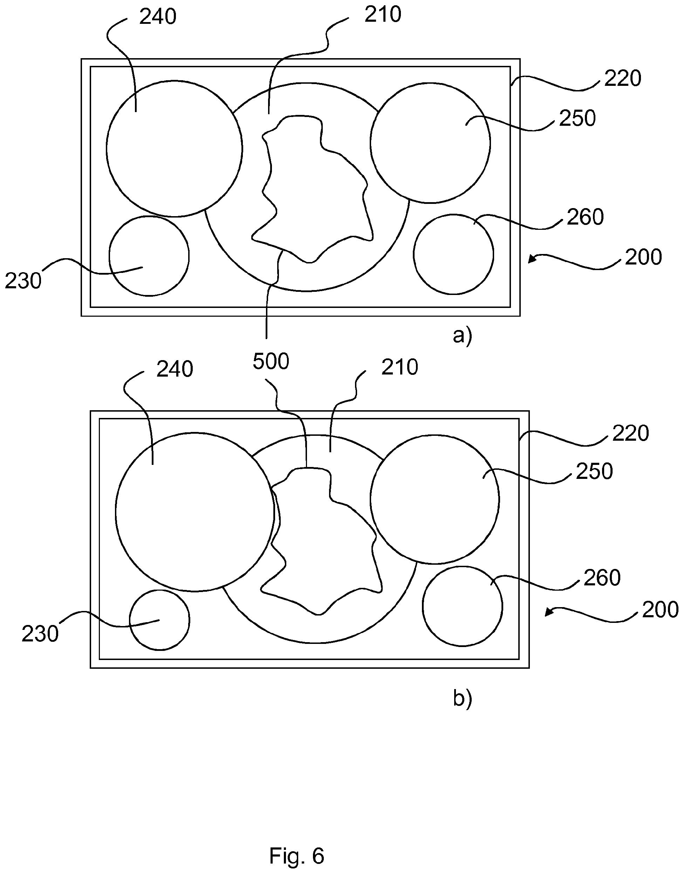

Ein der Erfindung entsprechendes Ausführungsbeispiel ist in Fig. 6 gezeigt. So zeigt Fig. 6 eine Bildanzeigeeinheit 200 mit einem Gesamtbild 220. Sowohl in Fig. 6a) als auch in Fig. 6b) werden Echtzeit-Videobildsignale des (nicht gezeigten) optischen Instruments permanent zentral in einem runden zentralen Bildfeld 210 des Gesamtbildes 220 angezeigt. Im Unterschied zu den vorbeschriebenen Ausführungsbeispielen sind das sagittale Nebenbild 240 und das coronare Nebenbild 250 im Bereich des zentralen Bildfeldes 220 angezeigt. Insbesondere sind das sagittale Nebenbild 240 und das coronare Nebenbild 250 vergrößert und überlappen das zentrale Bildfeld 220. Das axiale Nebenbild 230 und das Hilfsbild hingegen werden außerhalb des zentralen Bildfeldes 220 angezeigt und überlappen dieses nicht. Eine Vergrößerung des sagittalen Nebenbilds 240 und des coronaren Nebenbilds 250 wurde vorliegend dadurch bewirkt, dass die Signalverarbeitungseinheit 110 ausgebildet ist Instrumentenpositionssignale auszuwerten und in Abhängigkeit davon die Ausgangsbildsignale vergrößert zu generieren. Vorliegend hat sich der Chirurg mit dem Instrument 300 der Zielstruktur 500 genähert und ist kurz in dieser Position verharrt (erste Benutzeraktion). Fig. 6a) zeigt das Gesamtbild 220 nach einer ersten Benutzeraktion, Fig. 6b) nach einer zweiten Benutzeraktion, beispielsweise einer weiteren Annäherung an die Zielstruktur 500.

Auf dem zentralen Bildfeld 210 ist vorliegend eine Zielstruktur 500 eines (nicht gezeigten) Instruments 300 dargestellt, d.h. beispielsweise ein Operationsgebiet das während einer Operation ständig auf dem zentralen Bildfeld 210 zu sehen sein muss, also nicht verdeckt oder maskiert werden darf. Sowohl in Fig. 6a) als auch in Fig. 6b) werden die Nebenbilder 230, 240, 250 und das Hilfsbild derart zur Anzeige gebracht, dass die Zielstruktur 500 nicht verdeckt wird. Verdeckt wird hingegen ein Teil des zentralen Bildfelds 210. Im Ausführungsbeispiel der Fig. 6 ist die (nicht gezeigte) Signalverarbeitungseinheit 110 ausgebildet, die Zielstruktur 500 repräsentierende Signalteile der Echtzeit-VideoBildsignale zu erkennen und mittels einer Plausibilisierung zu verhindern, dass die Nebenbilder 230, 240, 250 und das Hilfsbild 260 durch eine Benutzeraktion derart vergrößert ausgegeben werden, dass die Zielstruktur 500 im zentralen Bildfeld 210 überdeckt wird. So ist es ausgehend von Fig. 6a) durch eine Benutzeraktion möglich, die Nebenbilder 230, 240, 250 und das Hilfsbild 260 weiter vergrößert darzustellen, da die Zielstruktur 500 im zentralen Bildfeld 210 mit einigem Abstand noch nicht überdeckt wird. Fig. 6b) zeigt die Situation nach einer derartigen Benutzeraktion. Ausgehend von Fig. 6b) wäre es durch eine Benutzeraktion unmöglich das sagittale Nebenbild 240 und das coronare Nebenbild 250 weiter zu vergrößern, da ansonsten die Zielstruktur 500 im zentralen Bildfeld 210 überdeckt würde.

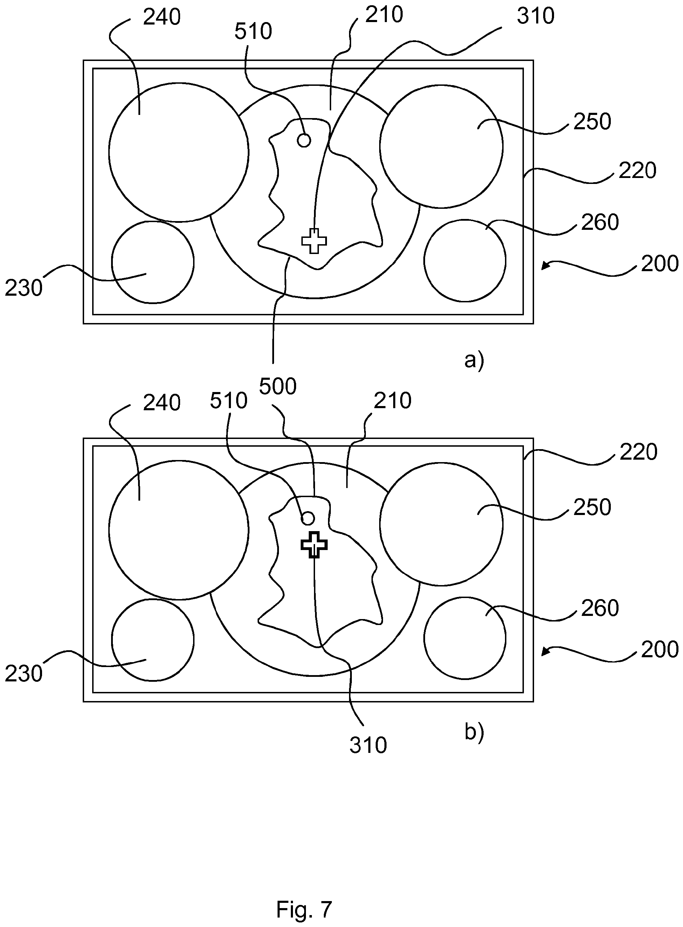

Fig. 7 zeigt schließlich ein Ausführungsbeispiel bei dem ein aufgenommenes Instrument repräsentierende Signalteile der Echtzeit-Videobildsignale erkannt werden. Hierzu ist die (nicht gezeigte) Signalverarbeitungseinheit 110 ausgebildet eine Mustererkennung in den Echtzeit-Videobildsignal durchzuführen und das so erkannte Instrument bzw. dessen Arbeitspunkt als zentrales Fadenkreuz 310 darzustellen. Das (nicht gezeigte) Instrument 300 ist also im zentralen Bildfeld 210 durch das zentrales Fadenkreuz 310 repräsentiert. Bei Annäherung des Instruments 300 an eine vorgebbare Position 510 wird das zentrale Fadenkreuz 310 optisch hervorgehoben. In Fig. 7a) ist ein zentrales Fadenkreuz 310 deutlich beabstandet von der vorgebbaren Position 510, d.h. das (nicht gezeigte) Instrument 300 ist auch im Operationsgebiet von der vorgebbaren Position 510, beispielsweise einem Ort an dem ein Schnitt vorgenommen werden soll, entfernt. Das zentrale Fadenkreuz 310 ist somit im zentralen Bildfeld 210 in normaler Strichstärke dargestellt. Wird nun das (nicht gezeigte) Instrument 300 durch den Chirurgen in die Nähe der vorgebbaren Position 510 gebracht, so wird zum einen das zentrale Fadenkreuz 310 im zentralen Bildfeld 210 in der Nähe der vorgebbaren Position 510 dargestellt, als auch das Fadenkreuz 310 im zentralen Bildfeld 210 in dickerer Strichstärke dargestellt.

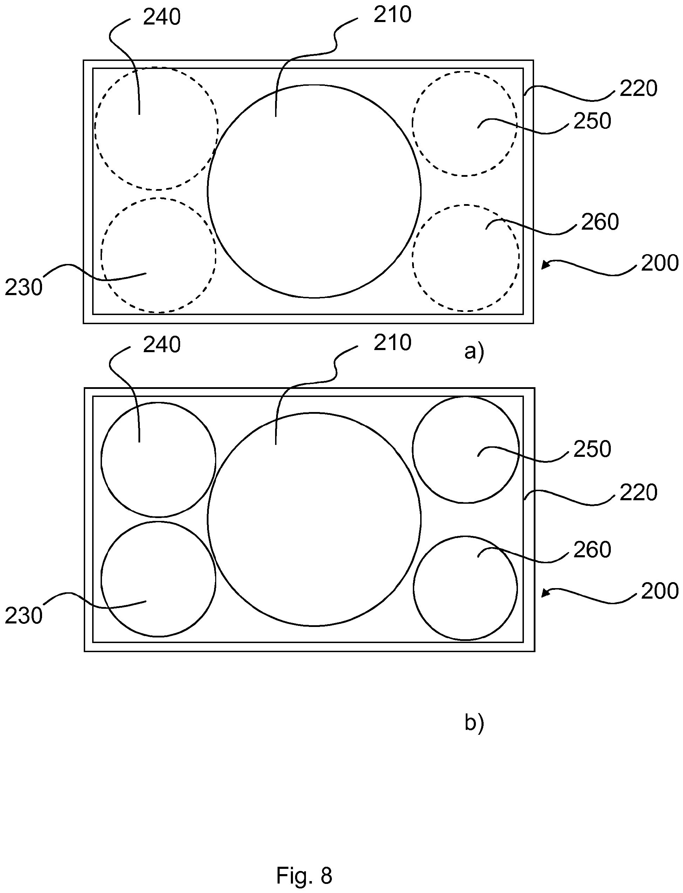

Fig. 8 zeigt eine schematische Darstellung eines Bildfeldes mit drei Nebenbildern und einem Hilfsbild angeordnet im Sinne einer dichtesten Kreispackung. Die Signalverarbeitungseinheit 110 ist ausgebildet, die Ausgangsbildsignale derart zu generieren, dass in der unteren linken Ecke des Gesamtbildes 220 ein axialer Schnitt, in der oberen linken Ecke ein sagittaler Schnitt und in der oberen rechten Ecke des Gesamtbildes ein coronarer Schnitt eines Tomographiebildes, in der unteren rechten Ecke des Gesamtbildes ein Hilfsbild 260 dargestellt wird. Das zentrale Bildfeld 210 und die Nebenbilder 230, 240, 250 und das Hilfsbild 260 weisen eine fixe Ausdehnung und Position auf dem Gesamtbild 220 auf. Die Signalverarbeitungseinheit 110 ist ausgebildet, die Ausgangsbildsignale derart zu generieren, dass die Nebenbilder 230, 240, 250, das Hilfsbild 260 und das zentrale Bildfeld 210 im Sinne einer dichtesten Kreispackung auf dem Gesamtbild 220 dargestellt werden. Die Nebenbilder 230, 240, 250, das Hilfsbild 260 und das zentrale Bildfeld 210 überlappen einander nicht.

### Bezugszeichenliste

- 100: Navigationsunterstützungssystem
- 110: Signalverarbeitungseinheit
- 112: Ausgang
- 113, 114: Eingang
- 115: Tomographiebilddaten
- 200: Bildanzeigeeinheit
- 210: Bildfeld
- 220: Gesamtbild
- 230, 240, 250: Nebenbilder
- 260: Hilfsbild
- 300: chirurgisches Instrument
- 310, 340: Fadenkreuz
- 400: optisches Instrument
- 500: Zielstruktur
- 510: Position
- V: vertikale Erstreckungsrichtung

## Patentansprüche

1. Navigationsunterstützungssystem (100) für medizinische Instrumente (300, 400), mit einer Signalverarbeitungseinheit (110), die einen Eingang (114) für Echtzeit-Videobildsignale von einem optischen Instrument (400), einen Eingang (113) für Instrumentenpositionssignale sowie Zugriff auf Tomographiebilddaten (115) besitzt und einen Ausgang (112) zum Ausgeben von Bildsignalen, wobei die Signalverarbeitungseinheit (110) ausgebildet ist, Ausgangsbildsignale aus den Echtzeit-Videobildsignalen, den Instrumentenpositionssignalen und den Tomographiebilddaten (115) zu generieren, **dadurch gekennzeichnet, dass**
die Signalverarbeitungseinheit (110) weiterhin ausgebildet ist, die Ausgangsbildsignale derart zu generieren, dass
- die Echtzeit-Videobildsignale permanent zentral auf einer an das Navigationsunterstützungssystem (100) angeschlossenen Bildanzeigeeinheit (200) in einem runden oder ovalen zentralen Bildfeld (210) eines Gesamtbildes (220) angezeigt werden, wobei sich das zentrale Bildfeld (210) wenigstens in einer Erstreckungsrichtung über mehr als die Hälfte des Gesamtbildes (220) erstreckt,
- aus Tomographiebilddaten (115) generierte Nebenbilder (230, 240, 250) in Abhängigkeit der Instrumentenpositionssignale neben dem zentralen Bildfeld (210) oder das zentrale Bildfeld (210) teilweise überlagernd dargestellt werden, und
- aus Tomographiebilddaten (115) generierte Nebenbilder (230, 240, 250) in Abhängigkeit der Instrumentenpositionssignale vergrößert dargestellt werden.

2. Navigationsunterstützungssystem nach Anspruch 1, **dadurch gekennzeichnet, dass** die Signalverarbeitungseinheit (110) weiterhin ausgebildet ist, die Ausgangsbildsignale derart zu generieren, dass sich das Gesamtbild (220) über die gesamte Fläche der Bildanzeigeeinheit (200) erstreckt.

3. Navigationsunterstützungssystem nach einem der vorangehenden Ansprüche, **dadurch gekennzeichnet, dass** die Signalverarbeitungseinheit (110) weiterhin ausgebildet ist, die Ausgangsbildsignale derart zu generieren, dass sich das zentrale Bildfeld (210) in vertikaler Erstreckungsrichtung (V) über mehr als die Hälfte des Gesamtbildes (220) erstreckt.

4. Navigationsunterstützungssystem nach einem der vorangehenden Ansprüche, **dadurch gekennzeichnet, dass** die Signalverarbeitungseinheit (110) ausgebildet ist, die Ausgangsbildsignale derart zu generieren, dass das Gesamtbild im Breitbildformat, insbesondere im Format 16:9 angezeigt wird.

5. Navigationsunterstützungssystem nach einem der vorangehenden Ansprüche, **dadurch gekennzeichnet, dass** die Signalverarbeitungseinheit (110) weiterhin ausgebildet ist, die Ausgangsbildsignale derart zu generieren, dass die Nebenbilder axiale (230) sagittale (240) und coronare (250) Schichten und/oder Schnitte sind.

6. Navigationsunterstützungssystem nach einem der vorangehenden Ansprüche, **dadurch gekennzeichnet, dass** die Signalverarbeitungseinheit (110) weiterhin ausgebildet ist, die Ausgangsbildsignale derart zu generieren, dass die Nebenbilder (230, 240, 250) nur in einem Navigationsmodus dargestellt und anderenfalls maskiert sind.

7. Navigationsunterstützungssystem nach einem der vorangehenden Ansprüche, **dadurch gekennzeichnet, dass** die Signalverarbeitungseinheit (110) weiterhin ausgebildet ist, die Ausgangsbildsignale derart zu generieren, dass die Position der Nebenbilder (230, 240, 250) im Gesamtbild (220) variabel ist.

8. Navigationsunterstützungssystem nach einem der vorangehenden Ansprüche, **dadurch gekennzeichnet, dass** die Signalverarbeitungseinheit (110) weiterhin ausgebildet ist, die Ausgangsbildsignale derart zu generieren, dass die Nebenbilder (230, 240, 250) auf Benutzeraktion hin im Bereich des zentralen Bildfeldes (220) angezeigt werden.

9. Navigationsunterstützungssystem nach einem der vorangehenden Ansprüche, **dadurch gekennzeichnet, dass** die Signalverarbeitungseinheit (110) weiterhin ausgebildet ist, die Ausgangsbildsignale derart zu generieren, dass wenn Nebenbilder (230, 240, 250) im Bereich des zentralen Bildfeldes (220) oder dieses überlappend angezeigt werden, dies derart erfolgt, dass keine Zielstrukturen/Abbildungen (500) eines Instruments (300) verdeckt werden.

10. Navigationsunterstützungssystem nach einem der vorangehenden Ansprüche, **dadurch gekennzeichnet, dass** die Signalverarbeitungseinheit (110) ausgebildet ist, ein aufgenommenes Instrument (300) repräsentierende Signalteile der Echtzeit-Videobildsignale zu erkennen.

11. Navigationsunterstützungssystem nach einem der vorangehenden Ansprüche, **dadurch gekennzeichnet, dass** die Signalverarbeitungseinheit (110) weiterhin ausgebildet ist, die Ausgangsbildsignale derart zu generieren, dass ein aufgenommenes Instrument (300) repräsentierende Signalteile der Echtzeit-Videobildsignale bei Annäherung des Instruments (300) an eine kritische Struktur und/oder Zielstruktur, insbesondere bei Annäherung an eine vorgebbare Position (510) optisch hervorgehoben werden.

12. Verfahren zum Betreiben eines Navigationsunterstützungssystems nach einem der vorangehenden Ansprüche mit dem Schritt:
- Generieren von Ausgangsbildsignalen aus Echtzeit-Videobildsignalen, Instrumentenpositionssignalen und Tomographiebilddaten derart, dass die Echtzeit-Videobildsignale permanent zentral auf einer an das Navigationsunterstützungssystem angeschlossenen Bildanzeigeeinheit in einem runden oder ovalen zentralen Bildfeld eines Gesamtbildes angezeigt werden, wobei sich das zentrale Bildfeld wenigstens in einer Erstreckungsrichtung über mehr als die Hälfte des Gesamtbildes erstreckt, wobei aus Tomographiebilddaten generierte Nebenbilder in Abhängigkeit der Instrumentenpositionssignale neben dem zentralen Bildfeld oder das zentrale Bildfeld teilweise überlagernd dargestellt werden und wobei aus Tomographiebilddaten (115) generierte Nebenbilder (230, 240, 250) in Abhängigkeit der Instrumentenpositionssignale vergrößert dargestellt werden.

## Claims

1. Navigation support system (100) for medical instruments (300, 400) comprising a signal processing unit (110) having an input (114) for real-time video image signals from an optical instrument (400), an input (113) for instrument position signals and access to tomography image data (115), and an output (112) for outputting image signals, wherein the signal processing unit (110) is configured for generating output image signals from the real-time video image signals, the instrument position signals and the tomography image data (115), **characterized in that**
the signal processing unit (110) is further configured for generating the output image signals in such a way that
- the real-time video image signals are displayed permanently centrally on an image display unit (200) connected to the navigation support system (100) in a round or oval central image field (210) of an overall image (220), wherein the central image field (210) extends at least in one extension direction over more than half of the overall image (220),
- secondary images (230, 240, 250) generated from tomography image data (115) are displayed in dependence on the instrument position signals next to or partially overlaying the central image field (210), and
- the secondary images (230, 240, 250) generated from tomography image data (115) are displayed enlarged in dependence on the instrument position signals.

2. Navigation support system according to claim 1, **characterized in that** the signal processing unit (110) is further configured for generating the output image signals in such a way that the overall image (220) extends over the entire area of the image display unit (200).

3. Navigation support system according to one of the preceding claims, **characterized in that** the signal processing unit (110) is further configured for generating the output image signals in such a way that the central image field (210) extends in the vertical direction of extension (V) over more than half of the overall image (220).

4. Navigation support system according to one of the preceding claims, **characterized in that** the signal processing unit (110) is configured for generating the output image signals in such a way that the overall image is displayed in widescreen format, particularly in the format 16:9.

5. Navigation support system according to one of the preceding claims, **characterized in that** the signal processing unit (110) is further configured for generating the output image signals in such a way that the secondary images are axial (230), sagittal (240) and coronary (250) layers and/or sections.

6. Navigation support system according to one of the preceding claims, **characterized in that** the signal processing unit (110) is further configured for generating the output image signals in such a way that the secondary images (230, 240, 250) are only shown in a navigation mode and are otherwise masked.

7. Navigation support system according to one of the preceding claims, **characterized in that** the signal processing unit (110) is further configured for generating the output image signals in such a way that the positions of the secondary images (230, 240, 250) in the overall image (220) are variable.

8. Navigation support system according to one of the preceding claims, **characterized in that** the signal processing unit (110) is further configured for generating the output image signals in such a way that the secondary images (230, 240, 250) are only shown in the area of the central image field (220) upon user action.

9. Navigation support system according to one of the preceding claims, **characterized in that** the signal processing unit (110) is further configured for generating the output image signals in such a way that if secondary images (230, 240, 250) are displayed in the region of the central image field (220) or overlapping it, this is done in such a way that no target structures/images (500) of an instrument (300) are concealed.

10. Navigation support system according to one of the preceding claims, **characterized in that** the signal processing unit (110) is configured for recognizing such signal parts of the real-time video image signals that represent a recorded instrument (300).

11. Navigation support system according to one of the preceding claims, **characterized in that** the signal processing unit (110) is configured for generating the output image signals in such a way that signal parts of the real-time video image signals that represent a recorded instrument (300) are highlighted visually upon the instrument (300) approaching a critical structure and/or a target structure, particularly upon approaching a pre-determinable position (510).

12. Method for operating a navigation support system according to one of the preceding claims with the step:
- generating output image signals from real-time video image signals, instrument position signals and tomography image data such that the real-time video image signals are permanently displayed centrally on an image display unit connected to the navigation support system in a round or oval central image field of an overall image, wherein the overall image extends in at least one direction of extension over more than half of the overall image, wherein secondary images generated from tomography image data (115) are displayed next to or partially overlaying the central image field in dependence on the instrument position signals and wherein secondary images (230, 240, 250) generated from tomography image data are displayed enlarged in dependence on the instrument position signals.

## Revendications

1. Système (100) d'assistance à la navigation pour des instruments (300, 400) médicaux, comprenant une unité (110) de traitement du signal, qui possède une entrée (114) pour des signaux d'image vidéo en temps réel d'un instrument (400) optique, une entrée (113) pour des signaux de position d'instrument, ainsi qu'un accès à des données (115) d'image tomographique et une sortie (112) d'émission de signaux d'image, l'unité (110) de traitement du signal étant constituée pour produire des signaux d'image de sortie à partir des signaux d'image vidéo en temps réel, des signaux de position d'instrument et des données (115) d'image tomographique, **caractérisé en ce que**
l'unité (110) de traitement du signal est constituée, en outre, pour produire les signaux d'image de sortie, de manière à ce que
- les signaux d'image vidéo en temps réel soient affichés en permanence de manière centrale sur une unité (200) d'affichage d'image raccordée au système (100) d'assistance à la navigation dans un champ (210) d'image central, circulaire ou ovale d'une image (220) d'ensemble, le champ (210) d'image central s'étendant au moins dans une direction d'étendue sur plus de la moitié de l'image (220) d'ensemble,
- des images (230, 240, 250) secondaires, produites à partir des données (115) d'image tomographique, soient représentées, en fonction des signaux de position d'instrument, à côté du champ (210) d'image central ou en étant superposées en partie au champ (210) d'image central et
- des images (230, 240, 250) secondaires, produites à partir de données (115) d'image tomographique, soient représentées agrandies en fonction des signaux de position d'instrument.

2. Système d'assistance à la navigation suivant la revendication 1, **caractérisé en ce que** l'unité (110) de traitement du signal est constituée, en outre, pour produire des signaux d'image de sortie, de manière à ce que l'image (220) d'ensemble s'étende sur toute la surface de l'unité (200) d'affichage d'image.

3. Système d'assistance à la navigation suivant l'une des revendications précédentes, **caractérisé en ce que** l'unité (110) de traitement du signal est constituée, en outre, pour produire les signaux d'image de sortie, de manière à ce que le champ (210) d'image central s'étende dans la direction (V) d'étendue verticale sur plus de la moitié de l'image (220) d'ensemble.

4. Système d'assistance à la navigation suivant l'une des revendications précédentes, **caractérisé en ce que** l'unité (110) de traitement du signal est constituée pour produire les signaux d'image de sortie, de manière à afficher l'image d'ensemble en grand format d'image, notamment en format 16.9.

5. Système d'assistance à la navigation suivant l'une des revendications précédentes, **caractérisé en ce que** l'unité (110) de traitement du signal est constituée, en outre, pour produire des signaux d'image de sortie, de manière à ce que les images secondaires soient des couches et/ou des coupes axiales (230), sagittales (240) et coronaires (250).

6. Système d'assistance à la navigation suivant l'une des revendications précédentes, **caractérisé en ce que** l'unité (110) de traitement du signal est constituée, en outre, pour produire les signaux d'image de sortie, de manière à ce que les images (230, 240, 250) secondaires ne soient représentées que dans un mode de navigation et, sinon, soient masquées.

7. Système d'assistance à la navigation suivant l'une des revendications précédentes, **caractérisé en ce que** l'unité (110) de traitement du signal est constituée, en outre, pour produire les signaux d'image de sortie, de manière à ce que la position des images (230, 240, 250) secondaires, dans l'image (220) d'ensemble, soit variable.

8. Système d'assistance à la navigation suivant l'une des revendications précédentes, **caractérisé en ce que** l'unité (110) de traitement du signal est constituée, en outre, pour produire les signaux d'image de sortie, de manière, sur réaction d'un utilisateur, à afficher les images (230, 240, 250) secondaires dans la partie du champ (220) d'image central.

9. Système d'assistance à la navigation suivant l'une des revendications précédentes, **caractérisé en ce que** l'unité (110) de traitement du signal est constituée, en outre, pour produire les signaux d'image de sortie, de manière à ce que, lorsque des images (230, 240, 250) secondaires sont affichées dans la partie du champ (220) d'image central ou le chevauchent, cela s'effectue de manière à ce qu'une structure cible/reproduction (500) d'un instrument (300) ne soit pas recouverte.

10. Système d'assistance à la navigation suivant l'une des revendications précédentes, **caractérisé en ce que** l'unité (112) de traitement du signal est constituée pour reconnaître des parties de signal, représentant un instrument (300) enregistré, des signaux d'image vidéo en temps réel.

11. Système d'assistance à la navigation suivant l'une des revendications précédentes, **caractérisé en ce que** l'unité (110) de traitement du signal est constituée, en outre, pour produire les signaux d'image de sortie, de manière à faire ressortir visuellement, lorsqu'un instrument (300) se rapproche d'une structure critique et/ou d'une structure cible, notamment lorsqu'il se rapproche d'une position (510) pouvant être donnée à l'avance, des parties de signal, représentant l'instrument (300) enregistré, des signaux d'image vidéo en temps réel.

12. Procédé pour faire fonctionner un système d'assistance à la navigation suivant l'une des revendications précédentes, comprenant les stades :
- production de signaux d'image de sortie à partir de signaux d'image vidéo en temps réel, de signaux de position d'instrument et de données d'image tomographique, de manière à ce que les signaux d'image vidéo en temps réel soient affichés en permanence de manière centrale sur une unité d'affichage d'image raccordée au système d'assistance à la navigation dans un champ d'image central, circulaire ou ovale d'une image d'ensemble, le champ d'image central s'étendant au moins dans une direction d'étendue sur plus de la moitié de l'image d'ensemble, des images secondaires produites à partir de données d'image topographique étant représentées, en fonction des signaux de position d'instrument, à côté du champ d'image central ou en étant partiellement superposées au champ d'image central et des images (230, 240, 250) secondaires, produites à partir de données (115) d'image tomographique, étant représentées agrandies en fonction des signaux de position d'instrument.
